# EUROPÄISCHE PATENTSCHRIFT

(11) **EP 4 136 412 B1**
(45) Veröffentlichungstag und Bekanntmachung des Hinweises auf die Patenterteilung: **28.01.2026**
(21) Anmeldenummer: 21719565.0
(22) Anmeldetag: 13.04.2021
(51) Int. Cl.: G01F 23/263, A24F 40/50, A61M 15/06, A61M 11/00, A61M 16/00, G01F 1/37, G01F 1/68, A61M 15/00

(54) **INHALATOR**
INHALER
INHALATEUR

(30) Priorität: 15.04.2020 DE 102020110258
(43) Veröffentlichungstag der Anmeldung: 22.02.2023
(73) Patentinhaber: Körber Technologies GmbH, 21033 Hamburg (DE)
(72) Erfinder: MIRDOGAN, Alp, 22761 Hamburg (DE); KESSLER, Marc, 22415 Hamburg (DE); HANNEKEN, Christian, 22299 Hamburg (DE)
(74) Vertreter: Müller Verweyen
(86) Internationale Anmeldenummer: PCT/EP2021/059538
(87) Internationale Veröffentlichungsnummer: WO 2021/209438

(56) Entgegenhaltungen:
- WO-A1-2017/192767
- CA-A1- 3 098 180
- TW-A- 201 826 953
- US-A- 5 363 842
- US-A1- 2017 318 861
- US-A1- 2019 289 919
- US-A1- 2020 338 285
- US-B2- 10 602 781
- US-B2- 9 854 841

## Beschreibung

Die vorliegende Erfindung betrifft einen Inhalator umfassend ein Gehäuse, einen sich zwischen mindestens einer Lufteinlassöffnung und einer Saugöffnung in dem Gehäuse erstreckenden Luftkanal, ein Verabreichungselement zum Vernebeln oder Verdampfen von dem Verabreichungselement zugeführter Flüssigkeit zur Beimischung zu in dem Luftkanal strömender Luft, eine elektronische Steuerungsvorrichtung, einen elektronischen Datenspeicher und ein Sensorsystem mit einer Durchflussmessvorrichtung zur Messung des Volumen- und/oder Massenstroms des durch den Luftkanal strömenden Luftstroms.

Ein derartiger Inhalator ist beispielsweise aus der EP 3 574 779 A2 bekannt.

Heutige elektronische Zigarettenprodukte und Inhalatoren dosieren den abzugebenden Wirkstoff über einen voreingestellten, nutzerunabhängigen Verabreichungsmechanismus aufweisend eine Verabreichungssteuerung und ein Verabreichungselement. Dabei aktiviert die Steuerung das Verabreichungselement beispielsweise infolge eines mittels eines Sensors gemessenen Unterdrucks während eines Inhalationszuges. Die abgegebene Wirkstoffmenge ist dabei im Wesentlichen durch die Aktivierungsdauer des Verabreichungselements festgelegt. Ein Verabreichungselement kann dabei ein Heizelement, ein Ultraschallvernebler, der mit Hilfe eines Piezoelements Flüssigkeit verdampft oder vernebelt, ein Gaskompressor, der einen Gasdruck aufbaut und dadurch Flüssigkeit durch eine Düse vernebelt oder verdampft, oder eine Verneblungsmembran, bei der durch hochfrequente Schwingung der Membran Flüssigkeit verdampft oder vernebelt wird, sein.

Da aktuell keinerlei Zusammenhang zwischen der Verabreichungssteuerung und der während eines Inhalationszugs durch den Inhalator gesaugten, nutzer-individuellen Luftmenge (z.B. unterscheidet sich die angesaugte Luftmenge bei einem starken oder schwachen Inhalationszug) besteht, ist die Dampfqualität, d.h. die Wirkstoffmenge pro Luftvolumen sowie die Tropfengrößenverteilung, im Wesentlichen abhängig vom individuellen Zugverhalten des Nutzers. Dies kann sowohl das reproduzierbare Raucherlebnis als auch eine definierte Wirkstoffabgabe in Bezug auf die Menge und den Abgabezeitpunkt in das inhalierte Luftvolumen negativ beeinflussen.

Aus der US 10,602,781 B2 ist ein Gerät zum Erzeugen von Aerosol bekannt, das einen Zerstäuber mit einem Heizer umfasst. Weiterhin umfasst das Gerät einen Sensor, mit dem eine Flussrate oder ein Druck ermittelt werden kann. Zum Einstellen der dem Heizer zugeführten Leistung werden die mittels des Senors ermittelten Werte mit Schwellwerten verglichen. Bei Über- oder Unterschreitung dieser Schwellwerte wird die für den Heizer bereitgestellte Leistung angepasst.

Die US 2019/0289919 A1 offenbart eine Kapsel für eine elektronische Verdampfungsvorrichtung mit einem Speicher, auf dem Informationen gespeichert sind, die zur Steuerung der Verdampfung genutzt werden.

Die US 2017/0318861 A1 offenbart ein Inhalationsgerät mit einem Heizer zur Verdampfung von Flüssigkeit. Es ist ferner eine Zugdetektionseinrichtung vorgesehen, mit der ein Zug an dem Inhalationsgerät durch einen Nutzer erkannt werden kann und infolgedessen der Heizer aktiviert wird.

Aus der US 9,854,841 B2 ist ein elektronischer Rauchartikel mit einem Flüssigkeitskapazitätssensor bekannt.

Die WO 2017/192767 A1 offenbart eine Tröpfchenabgabevorrichtung mit einer Einrichtung, die zur Messung der Aerosolabgabe eingerichtet ist.

Die US 5,363,842 A offenbart einen Inhalator, der den Erfolg oder Misserfolg einer Inhalatorbetätigung signalisieren kann.

Weiterhin zeigt die CA 3 098 180 A1 ein Inhalationsgerät, das charakteristische Muster in Echtzeit bei der Verwendung erfassen und messen kann. Das Inhalationsgerät kann in einem drahtlosen Kommunikationsmodus verwendet werden, um mit einem Display zu kommunizieren und die Nutzung des Inhalationssystems durch die Versuchsperson gleichzeitig mit der Inhalation zu beurteilen.

Aufgabe der vorliegenden Erfindung ist die Bereitstellung eines Inhalators mit verbesserter Reproduzierbarkeit sowohl des Rauch- bzw. Dampferlebnisses als auch der Wirkstoffdosierung.

Die Erfindung löst diese Aufgabe mit den Merkmalen der unabhängigen Ansprüche.

Erfindungsgemäß erfolgt eine Erfassung einer Mehrzahl von Luftstrommesswerten über einen Teil oder die gesamte Dauer eines Inhalationszugs. Anschließend erfolgt ein Vergleich der Mehrzahl von Luftstrommesswerten mit einem in dem Datenspeicher gespeicherten Zugprofil. Die Ausgabe eines Steuersignals erfolgt auf der Grundlage des Ergebnisses des Vergleichs der Mehrzahl von Luftstrommesswerten mit dem gespeicherten Zugprofil. Das gespeicherte Zugprofil kann dabei wie eine Kalibrierung gesehen werden. Die Luftstrommesswerte sind von der Durchflussmessvorrichtung in Abhängigkeit von der Zeit gemessene Luftvolumenstrom- und/oder Luftmassenstrom-Messwerte und/oder Druckänderungen in dem Bereich des Inhalators, durch den der Luftmassenstrom während des Inhalationszuges strömt. Ein Zugprofil im Sinne der Erfindung ist ein definierter idealer zeitlicher Verlauf eines Luft- oder Luft-Dampf-Volumenstroms (und/oder -Massenstroms), der während wenigstens eines Teils oder während des gesamten idealen Inhalationszuges des Nutzers durch den Inhalator strömt. Erfindungsgemäß erfolgt ein Vergleich zwischen dem vorgegebenen Zugprofil mit Messwerten, die während eines konkreten Inhalationszuges aufgenommen werden.

Die Erfindung stellt insbesondere eine Rückkopplung auf der Grundlage des gemessenen Luftstromprofils während eines Inhalationszuges, und bevorzugt auch der während des Inhalationszuges abgegebenen Wirkstoff-/Flüssigkeitsmenge, bereit. Aufgrund der bevorzugten Rückkopplung zwischen dem Luftstromprofil während eines Inhalationszuges, und bevorzugt auch der während des Inhalationszuges abgegebenen Wirkstoff-/Flüssigkeitsmenge, auf Basis des Ergebnis des Vergleichs mit einem idealen Zugprofil ist eine definierte und reproduzierbare Wirkstoffabgabe oder eine Anzeige an den Nutzer einer fehlerhaften Anwendung des Inhalators möglich. Durch die erfindungsgemäße Regelung passt sich der Inhalator quasi an einen Nutzer an, indem er den individuellen Atemzug eines Nutzers bei der Wirkstoff- bzw. Flüssigkeitsabgabe in den Luftstrom berücksichtigt.

Alternativ ist denkbar, dass der Inhalator ein fest vorgegebenes Wirkstoffabgabe-Profil aufweist und der Nutzer durch eine entsprechende Rückkopplung auf die Einhaltung eines dazu passenden Luftstrom- d.h. Inhalationsprofils trainiert wird, indem dem Nutzer Abweichungen entsprechend signalisiert werden.

Erfindungsgemäß löst bei einer definierten Abweichung der Luftstrommesswerte von dem gespeicherten Zugprofil das Steuersignal eine geeignete Maßnahme aus. Die geeignete Maßnahme umfasst vorzugsweise eine Anpassung der Ansteuerung des Heizelements (genauer des durch das Heizelement fließenden Heizstroms und/oder der Heizdauer und/oder einem Puls-Pause-Verhältnis der Ansteuerung), die Anpassung der Vibrationsgeschwindigkeit eines Piezoelements oder einer Verneblungsmembran oder die Anpassung der Austrittsgeschwindigkeit eines komprimierten Gases aus einer Düse durch Einstellen eines Gasdrucks, z.B. des Luftdrucks für die zu verabreichende Flüssigkeitsmenge, und/oder Signalisieren einer entsprechenden Information für einen Benutzer auf einer Signaleinrichtung.

Ausgehend von dem Ergebnis des Vergleichs zwischen den Durchflussmesswerten und dem Zugprofil können demnach verschiedene alternative Reaktionen erfolgen. Eine bevorzugte Möglichkeit besteht darin, dem Nutzer des Inhalators mittels einer geeigneten Signaleinrichtung zu signalisieren, dass beispielsweise die während des Inhalationszuges abgegebene Wirkstoffmenge nicht ausreichend war. Im Szenario eines zu starken Inhalationszuges und einer eventuell damit einhergehenden Überdosis kann die Verabreichung mithilfe der elektronischen Steuerung unterbunden werden, sodass das Auftreten von Medikamentnebenwirkungen vermieden oder im Notfall entsprechende Maßnahmen (z.B. Ruf des Notarztes oder Krankenwagens) eingeleitet werden können.

Eine weitere bevorzugte Möglichkeit besteht darin, dass ausgehend von dem Grad der Abweichung der Messwerte von dem Zugprofil die Wirkstoffabgabe in den Luft-/Luft-Dampf-Volumenstrom durch entsprechende Ansteuerung der Verabreichungsvorrichtung bzw. des Heizelements, des Piezoelements, der Verneblungsmembran oder des Luftkompressors angepasst wird. Dazu wird der durch das Heizelement fließende Heizstrom auf der Grundlage des Steuersignals angepasst. Wenn die Steuerung des Heizstroms vorteilhaft mittels digitaler Pulsweitenmodulation (PWM) mit zwei diskreten Zuständen erfolgt, können einer oder mehrere der folgenden Parameter zur Anpassung des Heizstroms verwendet werden: Dauer des AN-Zustands (Spannung liegt an); Dauer des AUS-Zustands (keine Spannung liegt an); das Verhältnis zwischen der Dauer des AN-Zustands und Dauer des AUS-Zustands (Puls-Pause-Verhältnis); andere Aktivierungs-/Deaktivierungsgrenzen, wie z.B. Widerstandsgrenzen des Heizers; Höhe des Pegels, d.h. der an das Heizelement angelegten Spannung, während des AN-Zustands.

Es ist auch möglich, dass beide zuvor beschriebenen Reaktionen durchgeführt werden. Beispielsweise kann zunächst versucht werden, die abgegebene Wirkstoffmenge per Verdampfungssteuerung anzupassen, d.h. Anpassung der Ansteuerung des Heizelements bzw. des durch das Heizelement fließenden Heizstroms. Falls dies während des jeweiligen Inhalationszuges nicht mehr möglich ist, z.B. infolge einer zu großen Abweichung der Messwerte von dem Zugprofil, kann dem Nutzer zum Ende und/oder nach dem Inhalationszug die Abweichung von dem Zugprofil derart signalisiert werden, dass der Nutzer seinen Inhalationszug anpassen kann. Sollte wiederum eine solche Anpassung des Inhalationszuges nicht ausreichen, kann dem Nutzer eine Fehldosierung signalisiert werden.

Die erfindungsgemäß bevorzugte Rückkopplung kann vorteilhaft durch einen geeigneten Regelkreislauf, insbesondere umfassend eine digitale elektronische Steuerungsvorrichtung (beispielsweise Mikroprozessor), einen elektronischen, insbesondere nichtflüchtigen Datenspeicher und ein Sensorsystem oder ein Teil davon, realisiert sein. Der elektronische Datenspeicher ist eingerichtet, wenigstens ein Zugprofil dauerhaft zu speichern. Das Sensorsystem weist eine Durchflussmesseinrichtung auf, die eingerichtet ist, den zeitlichen Verlauf des Luft-/Luft-Dampf-Volumenstroms während eines konkreten Inhalationszuges zu messen. Die elektronische Steuerungsvorrichtung ist zweckmäßigerweise mit dem Sensorsystem, dem Datenspeicher, dem Heizelement und einer ggf. vorhandenen Signaleinrichtung verbunden.

Die Durchflussmessvorrichtung kann vorteilhaft eine Differenzdruckmessvorrichtung sein, was eine zuverlässige Messung des Luftvolumenstroms (oder Luftmassenstroms) mit einfachen Mitteln in einem mobilen Inhalator ermöglicht. Die Differenzdruckmessvorrichtung umfasst in dieser Ausführungsform einen ersten Drucksensor, der zur Messung des Umgebungs-Luftdrucks des Inhalators angeordnet ist, und einen zweiten Drucksensor, der zur Messung des Luftdrucks im Luftkanal des Inhalators angeordnet ist. Durch Kenntnis des Querschnitts des Luftkanals am Messort des zweiten Drucksensors und der zwischen dem ersten und zweiten Drucksensor gemessenen Druckdifferenz kann der Luftvolumenstrom durch den Inhalator bestimmt werden.

In einer anderen Ausführungsform kann die Durchflussmessvorrichtung vorteilhaft eine Hitzdrahtmessvorrichtung (thermisches Anemometer) sein. Dabei wird mindestens ein in dem Luftkanal des Inhalators angeordnetes Drahtelement elektrisch erhitzt. Durch die Umströmung des Drahtelements findet ein Wärmetransport in die strömende Luft statt, d.h. das Drahtelement wird gekühlt. Durch die Messung des von der Temperatur abhängigen elektrischen Widerstands kann so die Strömungsgeschwindigkeit, und damit der Volumenstrom, der durch den Luftkanal strömenden Luft bestimmt werden.

Mittels der Durchflussmessvorrichtung ist auch eine Bestimmung des Luft-Volumenstroms im zeitlichen Verlauf möglich. Dadurch ergibt sich vorteilhaft ein "Puff"-Profil oder gemessenes Zugprofil des gesamten Zuges.

Vorzugsweise weist das Sensorsystem einen Flüssigkeitsmengensensor zur Erfassung der während eines Inhalationszuges von einer Verdampfungsvorrichtung abgegebenen Flüssigkeitsmenge auf. Dies erlaubt eine genauere Anpassung der zu verdampfenden Flüssigkeitsmenge an das jeweilige Zugprofil des Konsumenten.

In einer vorteilhaften Ausführungsform ist der Flüssigkeitsmengensensor ein Luftfeuchtesensor. Der Luftfeuchtesensor umfasst vorzugsweise zwei Feuchtemesselemente, die zur Messung der Luftfeuchte in dem Luftkanal des Inhalators angeordnet sind. Das erste Feuchtemesselement ist im Bereich des Lufteinlasses oder im Luftkanal stromaufwärts von dem Heizelement angeordnet und misst einen Referenzwert der Luftfeuchte vor der Beimischung von Flüssigkeitsdampf. Das zweite Feuchtemesselement ist in dem Luftkanal stromabwärts von dem Heizelement, beispielsweise im Bereich des Mundstücks, angeordnet und misst die Luftfeuchte direkt vor dem Einatmen des Luft-Aerosol-Dampf-Gemisches durch den Anwender. Bei einer bekannten Menge von Wasser in der zu verdampfenden Flüssigkeit und einer Kalibrierung des zweiten Feuchtemesselements kann aus dem Anstieg der Luftfeuchte, d.h. der Differenz der Messwerte aus dem zweiten und dem ersten Feuchtemesselement, die verdampfte Flüssigkeitsmenge bestimmt und im Weiteren berücksichtigt werden.

Ein aufgrund differenzieller Destillation sich ändernder Wasseranteil in der Flüssigkeit während der Entleerung der Kartusche kann vorteilhaft durch Bestimmung der Restflüssigkeitsmenge im Flüssigkeitstank mittels eines Füllstandssensors (siehe unten) oder Abschätzung durch das Zählen der Züge in Verbindung mit der Messung der Zugdauer kompensiert werden.

Aus der ermittelten Restflüssigkeitsmenge kann aufgrund der Kenntnis der differenziellen Destillation der aktuelle Wasseranteil berechnet und auf dieser Grundlage der gemessene Wert der Luftfeuchte korrigiert werden. Zudem kann aus der ermittelten oder abgeschätzten verdampften Flüssigkeitsmenge unter Berücksichtigung des Anteils des Wirkstoffs in der Flüssigkeit die verdampfte Wirkstoffmenge ermittelt werden.

Das Sensorsystem weist vorteilhaft einen Füllstandssensor zur Erfassung einer Restflüssigkeitsmenge in einem Flüssigkeitstank des Inhalators auf. Der Füllstandssensor kann beispielsweise ein kapazitiver Sensor sein. Aus dem mit dem Füllstandssensor gemessenen Füllstand der Flüssigkeit im Flüssigkeitstank kann die Restmenge an Flüssigkeit in dem Flüssigkeitstank einfach ermittelt werden. Die elektronische Steuerungsvorrichtung ist vorzugsweise zur Feststellung einer Dauer bis zu einem erforderlichen Kartuschenwechsel auf der Grundlage der mittels des Füllstandssensors gemessenen Restflüssigkeitsmenge eingerichtet, beispielsweise unter Berücksichtigung eines in dem Datenspeicher gespeicherten bisherigen Nutzungsprofils.

Alternativ kann der Füllstandssensor auf einer Absorptionsmessung beruhen. Dazu werden eine Lichtquelle mit einer definierten Wellenlänge, etwa eine LED oder ein Laser, und ein lichtempfindliches Element (Photoelement), etwa eine Photodiode, in oder mit Bezug zu dem Luftkanal derart angeordnet, dass der durch den Luftkanal strömende Luftstrom den Bereich zwischen Lichtquelle und lichtempfindlichem Element durchströmt. Die Wellenlänge des von der Lichtquelle emittierten Lichts ist derart gewählt, dass der durch den Heizer in die Gasphase verdampfte Wirkstoff einen hohen Absorptionsquerschnitt bei dieser für den Wirkstoff spezifischen Wellenlänge aufweist (z.B. im IR- oder UV-Bereich). Beim Durchströmen der mit dem Wirkstoff angereicherten Luft misst das Photoelement die Absorption (Abschwächung) des von der Lichtquelle emittierten Lichts. Der gemessene Absorptionswert im zeitlichen Verlauf ist in Verbindung mit der Bestimmung des Luftvolumenstroms durch den Luftkanal proportional zu der in den Luftstrom verdampften Wirkstoffmenge.

Die Signaleinrichtung kann beispielsweise eine oder mehrere LEDs, eine Digitalanzeige, ein Display, einen haptischen Signalgeber und/oder einen akustischen Signalgeber umfassen. Die elektronische Steuerungsvorrichtung ist eingerichtet, die Messwerte des Sensorsystems, genauer der Durchflussmessvorrichtung, mit dem wenigstens einen Zugprofil zu vergleichen. Ist das Ergebnis des Vergleichs eine Abweichung zwischen den Messwerten und dem Zugprofil, die einen bestimmten Schwellwert überschreitet, so ist die elektronische Steuerungsvorrichtung eingerichtet, das Heizelement und/oder die Signaleinrichtung derart anzusteuern, dass eine vorbestimmte Reaktion in Abhängigkeit von der bestimmten Abweichung erfolgt. Eine vorbestimmte Reaktion kann beispielsweise eine Verlängerung oder Verkürzung der Heizdauer des Heizelements zur Änderung der Verdampfung bzw. eine Änderung des Puls-Pause-Verhältnisses und damit der Abgabe des Wirkstoffs in den Luft-/Luft-Dampf-Volumenstrom sein. Eine vorbestimmte Reaktion kann beispielsweise auch eine per Signaleinrichtung angezeigte Anweisung an den Nutzer sein, seinen Inhalationszug anzupassen. Alternativ kann dem Nutzer angezeigt werden, dass eine Fehldosierung des Wirkstoffs erfolgt ist.

Insbesondere für die Anwendung der Erfindung im medizinischen Bereich kann das Zugprofil vorab in einer Initialisierungsprozedur beispielsweise in Abhängigkeit von einer Serie von Inhalationszügen eines Patienten ermittelt werden, d.h. es wird ein nutzer- bzw. patientenindividuelles Zugprofil bestimmt. Die nachfolgend beschriebene Initialisierungsprozedur ist nicht auf medizinische Anwendungen beschränkt, sondern kann auch bei nicht-medizinischen Anwendungen, beispielsweise Genussanwendungen, eingesetzt werden.

Dazu können mehrere Inhalationszüge ohne Wirkstoffabgabe durch Verdampfen am Heizelement vor der eigentlichen Anwendung gemessen werden ("trockene Züge"). Es ist auch denkbar, dass ein oder mehrere Atemluftstöße, z.B. durch Lungenvolumen- und/oder -funktionsmessungen, durchgeführt werden. Derartige Messungen könnten beispielsweise extern durch einen Arzt oder mit dem Inhalator selbst durchgeführt werden. Extern aufgenommene Daten können über eine Kommunikationseinrichtung des Inhalators (z.B. drahtlos beispielsweise auf der Grundlage von Bluetooth, WLAN, RFID, ZigBee, optisch oder kabelgebunden) an die elektronische Steuerungsvorrichtung und/oder den Datenspeicher übertragen werden.

Durch einen Vergleich mit einem theoretischen Zugprofil kann das individuelle Zugprofil des Patienten ermittelt werden. Dieses (patienten-)individuelle Zugprofil wird in dem elektronischen Speicher gespeichert. Die elektronische Steuerungsvorrichtung vergleicht bei der eigentlichen Benutzung des Inhalators mit Wirkstoffabgabe dieses (patienten-)individuelle Zugprofil mit den Messwerten des aktuellen Inhalationszuges und reagiert wie oben beschrieben entsprechend darauf.

Das (patienten-)individuelle Zugprofil kann auch die für den Patienten gewünschte Wirkstoffmenge berücksichtigen. Diese Wirkstoffmenge kann beispielsweise durch einen Arzt oder einen Apotheker (z.B. ausgehend von einem digitalen Rezept) vorgegeben und mittels der oben genannten Kommunikationseinrichtung an die elektronische Steuerungsvorrichtung und/oder den Datenspeicher übertragen werden.

Vorzugsweise können Nutzerdaten in dem Datenspeicher gespeichert werden. Beispielsweise können die Häufigkeit der Nutzung und/oder die Anzahl erfolgreicher Inhalationszüge in Abhängigkeit von Datum und Uhrzeit gespeichert werden. Durch Auslesen dieser Daten kann ein Arzt die (zeitlich) korrekte Anwendung des Inhalators und Applikation des Wirkstoffs überwachen, den Therapieplan des Patienten ggf. anpassen und die Daten bei der weiteren Diagnostik und Therapie berücksichtigen.

Weiterhin ist es möglich, dass die vorgenannten Nutzerdaten und/oder das Zugprofil über die Kommunikationseinrichtung zusätzlich oder alternativ zum Datenspeicher in einem Cloudspeicher abgelegt werden. In diesem Fall kann ein Arzt die Anwendung des Inhalators bei Zugriff auf die Daten im Cloudspeicher fernüberwachen.

Vorzugsweise können gespeicherte Daten an ein elektronisches Mobilgerät, z.B. ein Smartphone, Laptop, Smartwatch oder Tablet-PC, mit Hilfe der Kommunikationseinrichtung übertragen werden. In diesem Fall kann der Nutzer selbst die Anwendung des Inhalators mit Hilfe des Mobilgeräts überwachen. Auch kann er sich Terminerinnerungen in dem Mobilgerät einrichten, falls der Inhalator zu bestimmten Uhrzeiten oder in wiederkehrenden Intervallen angewendet werden soll.

Vorteilhaft kann bspw. bei klinischen Studien ab Phase I die regelmäßige Anwendung und damit eine lückenlose Dokumentation der Inhalatoranwendung sichergestellt werden. Auch kann der Nutzer bei der Übertragung der Daten an ein elektronisches Mobilgerät sein persönliches Empfinden vor und nach einer Anwendung des Inhalators notieren. Dadurch kann beispielsweise ein kausaler Zusammenhang zwischen bestimmten körperlichen Reaktionen und der Anwendung des Inhalators bzw. des damit applizierten Wirkstoffs engmaschig dokumentiert werden. Die damit gewonnenen Informationen z.B. zur Verträglichkeit eines Wirkstoffs können beispielsweise auch für den Arzt bei der Therapie einer Krankheit mit einem bereits zugelassenen Wirkstoff relevant sein.

Eine kontrollierte und reproduzierbare Wirkstoffabgabe ist eine Voraussetzung für die Verwendung der Erfindung bzw. der erfindungsgemäßen Regelung des durch das Heizelement fließenden Heizstroms im Bereich der medizinischen Anwendung. Mit anderen Worten ist die vorliegende Erfindung die Basis für die Erschließung eines vollkommen neuen Anwendungsbereiches.

Die Erfindung löst somit das Problem einer definierten Wirkstoff- bzw. Flüssigkeitsabgabe pro Luftvolumen bei einem zeitlich veränderlichen Luftstrom.

Die Erfindung wird im Folgenden anhand bevorzugter Ausführungsformen unter Bezugnahme auf die beigefügten Figuren erläutert.

Dabei zeigt
- Fig. 1: einen Längsschnitt durch einen Inhalator;
- Fig. 2: eine schematische Darstellung einer elektronischen Anordnung des Inhalators mit einem Sensorsystem;
- Fig. 3: einen Querschnitt durch einen Flüssigkeitstank mit einem mittig innenliegenden Luftkanal;
- Fig. 4: ein Ablaufschema für eine Nutzung eines Inhalators; und
- Fig. 5: ein Ablaufschema für eine Initialisierung eines Inhalators.

Der Inhalator 10, hier ein elektronisches Zigarettenprodukt, umfasst ein Gehäuse 11, in dem ein Luftkanal 30 zwischen mindestens einer Lufteinlassöffnung 31, und einer Luftauslassöffnung 24 an einem Mundabschnitt 32 des Inhalators 10 vorgesehen ist. Wenn der Konsument an dem Mundabschnitt 32 zwecks Inhalation zieht, wird dadurch der Inhalator 10 mit einem Unterdruck beaufschlagt und eine Luftströmung 34 wird in dem Luftkanal 30 erzeugt.

Der Inhalator 10 weist vorteilhaft einen Flüssigkeitsspeicher 18, einen elektrischen Energiespeicher 14, eine Verdampfungsvorrichtung

20 mit einem elektrischen Widerstands-Heizelement 21, eine elektrische Anordnung 22 und ein Sensorsystem 33 auf. Der Flüssigkeitsspeicher 18 ist vorteilhaft in einer Verbrauchseinheit 17 des Inhalators 10 angeordnet. Die Verbrauchseinheit 17 kann vorteilhaft als auswechselbare Kartusche ausgebildet sein.

Der elektrische Energiespeicher 14 ist vorteilhaft in einem Basisteil 16 des Inhalators 10 angeordnet. Der Energiespeicher 14 kann insbesondere eine elektrochemische Einweg-Batterie oder ein wiederaufladbarer elektrochemischer Akku, beispielsweise ein Lithium-Ionen-Akku, sein. Vorzugsweise ist der Energiespeicher 14 in einem von dem Mundabschnitt 32 entfernten Teil des Inhalators 10 angeordnet. Die Verbrauchseinheit 17 ist vorteilhaft zwischen dem Energiespeicher 14 und dem Mundabschnitt 32 angeordnet.

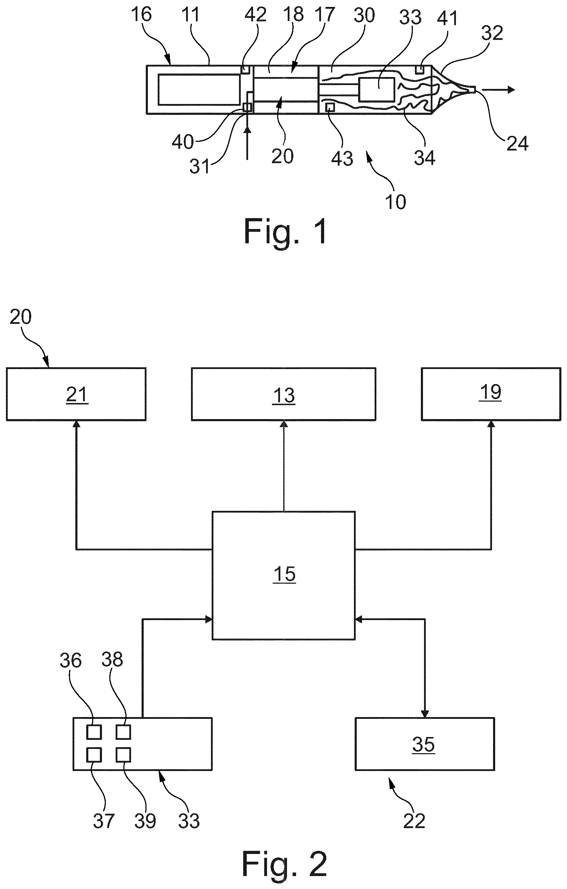

Die elektrische Anordnung 22 des Inhalators 10, die in Figur 2 schematisch dargestellt ist, weist das elektrische Widerstands-Heizelement 21, eine digitale elektronische Steuerungsvorrichtung 15 und einen elektronischen Datenspeicher 35 auf. Die elektronische Steuerungsvorrichtung 15 ist eine digitale Datenverarbeitungseinrichtung und umfasst vorzugsweise einen Mikroprozessor und/oder einen Microcontroller. Die elektrische Anordnung 22 kann vorzugsweise eine Kommunikationseinrichtung 13 und/oder eine Signaleinrichtung 19 aufweisen. Die Kommunikationseinrichtung 13 ist drahtlos, beispielsweise auf der Grundlage von Bluetooth, WLAN, RFID, ZigBee, optisch oder kabelgebunden, und vorzugsweise zur Kommunikation mit einem mobilen elektronischen Gerät, etwa einem Mobiltelefon oder Smartphone, einem externen Computer und/oder einem Cloudspeicher eingerichtet. Die Signaleinrichtung 19 umfasst optische, akustische und/oder haptische Signalelemente, beispielsweise eine oder mehrere LEDs, eine Digitalanzeige, ein Display, einen haptischen Signalgeber und/oder einen akustischen Signalgeber.

Teile der elektrischen Anordnung 22 sind vorzugsweise in dem Basisteil 16 angeordnet, beispielsweise Steuerungsvorrichtung 15, Datenspeicher 35, ggf. Kommunikationseinrichtung 13 und/oder ggf. Signaleinrichtung 19. Teile der elektrischen Anordnung 22 können in der Verbrauchseinheit 17 angeordnet sein, beispielsweise das Heizelement 21. In anderen Ausführungsformen ist das Heizelement 21 in dem Basisteil 16 angeordnet.

Die durch die Einlassöffnung 31 angesaugte Luft wird in dem Luftkanal 30 zu der, durch die, oder an der Verdampfungsvorrichtung 20 vorbei geleitet. Die Verdampfungsvorrichtung 20 ist mit dem Flüssigkeitsspeicher 18 verbunden oder verbindbar, in dem eine Flüssigkeit oder ein Flüssigkeitsgemisch gespeichert ist. Die Verdampfungsvorrichtung 20 verdampft Flüssigkeit, die ihr aus dem Flüssigkeitsspeicher 18 zugeführt wird, und gibt die verdampfte Flüssigkeit als Aerosol/Dampf in den Luftstrom 34 zu.

Das Sensorsystem 33 umfasst vorteilhaft einen in dem Luftkanal 30, oder in Strömungsverbindung dazu, angeordneten Druck- oder Strömungsschalter 36, der beispielsweise bei Unterschreiten einer vordefinierten Druckschwelle in dem Luftkanal 30 auslöst. Die Steuerungsvorrichtung 15 kann auf der Grundlage eines von dem Druck- oder Strömungsschalters 36 ausgegebenen Signals feststellen, dass ein Konsument am Mundabschnitt 32 des Zigarettenprodukts 10 zieht, um zu inhalieren. Die Strömungsmessvorrichtung 37 (siehe unten) kann die Funktion des Strömungsschalters 36 ausführen, oder es ist ein separater Strömungsschalter 36 vorhanden. Die Steuerungsvorrichtung 15 steuert dann die Verdampfungsvorrichtung 20 an, um Flüssigkeit aus dem Flüssigkeitsspeicher 18 als Aerosol/Dampf in den Luftstrom 34 zuzugeben.

Die in dem Flüssigkeitsspeicher 18 gespeicherte, zu dosierende Flüssigkeit 50 ist beispielsweise eine Mischung umfassend einen oder mehrere der folgenden Bestandteile: 1,2-Propylenglykol, Glycerin, Wasser, mindestens ein Aroma (Flavour), optional ein Wirkstoff, beispielsweise Nikotin.

Die Verdampfungsvorrichtung 20 umfasst mindestens einem Widerstands-Heizelement 21, und kann ein nicht gezeigtes Dochtelement zum Zuführen von Flüssigkeit aus dem Flüssigkeitsreservoir 18 zu dem Heizelement 21 aufweisen. Aufgrund des Ohm'schen Widerstands führt ein Stromfluss durch das elektrisch leitende Heizelement 21 zu einer Erhitzung desselben und daher zu einer Verdampfung von mit dem Heizelement 21 in Kontakt stehender Flüssigkeit. Auf diese Weise erzeugter Dampf/Aerosol entweicht aus der Verdampfungsvorrichtung 20 und wird der Luftströmung 34 beigemischt, siehe Figur 1. Die Verdampfungstemperatur liegt abhängig von der zu verdampfenden Flüssigkeit vorzugsweise im Bereich zwischen 100°C und 450°C, weiter bevorzugt zwischen 150°C und 350°C, noch weiter bevorzugt zwischen 190°C und 290°C.

Der Datenspeicher 35 ist vorteilhaft nichtflüchtig und dient beispielsweise zum Speichern von die Verbrauchseinheit 17 betreffender Information bzw. Parameter. Der Datenspeicher 35 kann Teil der elektronischen Steuerungsvorrichtung 15 sein. In dem Datenspeicher 35 ist vorteilhaft Information zur Zusammensetzung der in dem Flüssigkeitsspeicher 18 gespeicherten Flüssigkeit, Information zum Verdampfungsprofil, insbesondere zur Leistungs-/Temperatursteuerung, Daten zur Zustandsüberwachung bzw. Systemprüfung, beispielsweise Dichtigkeitsprüfung, Daten betreffend Kopierschutz und Fälschungssicherheit, eine ID zur eindeutigen Kennzeichnung der Verbrauchseinheit 17, Seriennummer, Herstelldatum, Ablaufdatum, Zugzahl (Anzahl der Inhalationszüge durch den Konsumenten) und/oder Nutzungszeit gespeichert.

Das Sensorsystem 33 weist eine Durchflussmessvorrichtung 37 auf, die hier als Differenzdruckmessvorrichtung ausgebildet ist. Die Durchflussmessvorrichtung 37 weist einen ersten Drucksensor 42 auf (siehe Figur 1), der so angeordnet ist, dass er zur Messung des Atmosphärenluftdrucks außerhalb des Gehäuses 11 des Inhalators 10 eingerichtet ist. Beispielsweise kann eine Messöffnung in dem Gehäuse 11 vorgesehen sein, der den ersten Drucksensor 42 strömungsleitend mit der Atmosphäre zu verbinden. Die Durchflussmessvorrichtung 37 weist des Weiteren einen zweiten Drucksensor 43 auf (siehe Figur 1), der so angeordnet ist, dass er zur Messung des in dem Luftkanal 30 herrschenden Luftdrucks eingerichtet ist. Die elektronische Steuerungsvorrichtung 15 kann durch Kenntnis des Querschnitts des Luftkanals am Messort des zweiten Drucksensors 43 und der zwischen dem ersten und zweiten Drucksensor 42, 43 gemessenen Druckdifferenz den Luftvolumenstrom durch den Inhalator 10 berechnen und durch zeitlich wiederholte Messung ein Zugprofil, d.h. den Luftvolumenstrom über der Zeit, bestimmen.

Das Sensorsystem 33 weist einen Dampfmengensensor 38 zur Erfassung der während eines Inhalationszuges von der Verdampfungsvorrichtung 20 verdampften und abgegebenen Flüssigkeits- bzw. Dampfmenge auf. Der Dampfmengensensor 38 ist hier ein Luftfeuchtesensor. Der Dampfmengensensor 38 weist ein erstes Feuchtemesselement 40 auf (siehe Figur 1), das in dem Luftkanal 30 stromaufwärts von dem Heizelement 21, beispielsweise im Bereich der Lufteinlassöffnung 31 angeordnet ist. Der Dampfmengensensor 38 weist des Weiteren ein zweites Feuchtemesselement 41 auf (siehe Figur 1), das in dem Luftkanal 30 stromabwärts von dem Heizelement, beispielsweise im oder im Bereich des Mundabschnitts 32 angeordnet ist. Bei einer bekannten Menge von Wasser in der zu verdampfenden Flüssigkeit und einer Kalibrierung des zweiten Feuchtemesselements 41 kann aus dem Anstieg der Luftfeuchte, d.h. der Differenz der Messwerte von dem zweiten Feuchtemesselement 41 und dem ersten Feuchtemesselement 40, die verdampfte Flüssigkeits- bzw. Dampfmenge von der elektronischen Steuerungsvorrichtung 15 bestimmt werden.

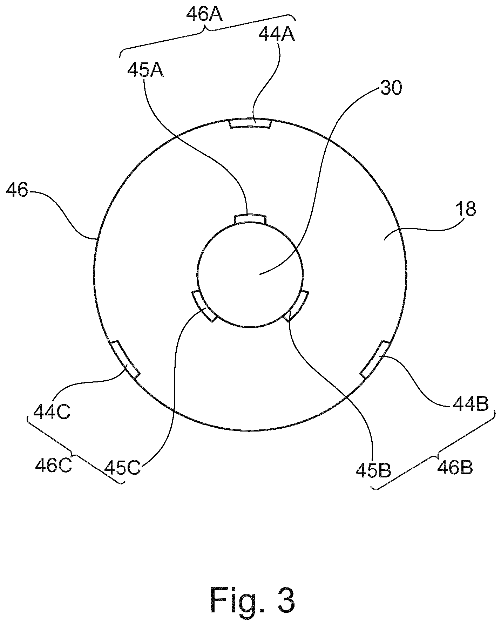

Der Flüssigkeitstank 18 ist vorzugsweise länglich mit mittig innenliegendem Luftkanal 30 (Figur 3 zeigt einen Schnitt quer zur Längsachse L durch den Flüssigkeitstank 18). Die in Figur 3 der Übersichtlichkeit wegen nicht gezeigte Verdampfungsvorrichtung 20 verdampft aus dem Flüssigkeitstank 18 zugeführte Flüssigkeit und gibt diese als Dampf/Aerosol an die durch den innenliegenden Luftkanal 30 strömende Luftströmung ab.

Das Sensorsystem 33 weist vorzugsweise einen Füllstandssensor 39 zur Erfassung einer Restflüssigkeitsmenge in dem Flüssigkeitstank 18 des Inhalators 10 auf. Der Füllstandssensor 39 ist hier ein kapazitiver Sensor und weist mindestens ein Paar von Elektroden 44A, 45A; 44A, 45B; 44C, 45C auf (siehe Figur 3), die an gegenüberliegenden Wänden 46, 47 des Flüssigkeitstanks 18 angeordnet sind. Die Elektroden 44A, 45A; 44A, 45B; 44C, 45C werden vorzugsweise von metallisierten Bereichen, insbesondere metallischen (Längs-)Streifen gebildet, die entlang der Längsachse des Flüssigkeitstanks 18 durchgehend verlaufen. Vorteilhaft ist mindestens eine erste Elektrode 44A, 44B, 44C an der beispielsweise zylindrischen Außenwand 46 des Flüssigkeitstanks 18 und mindestens eine zweite Elektrode 45A, 45B, 45C an der beispielsweise zylindrischen Innenwand 46 des Flüssigkeitstanks 18 (und/oder an der beispielsweise zylindrischen Außenwand des Luftkanals 30) angeordnet.

Der Füllstandssensor 39 weist vorzugsweise eine Mehrzahl von Elektrodenpaaren, beispielsweise mindestens oder genau drei Elektrodenpaare 44A, 45A; 44A, 45B; 44C, 45C auf. Die Elektrodenpaare 44A, 45A; 44A, 45B; 44C, 45C sind vorteilhaft unter gleichen Winkelabständen, in Figur 3 beispielsweise unter 120°, bezogen auf die Mittelachse des Flüssigkeitstanks 18, wechselseitig zueinander angeordnet. Die ersten Elektroden 44A, 44B, 44C und/oder die zweiten Elektroden 45A, 45B, 45C können jeweils zu einer durchgehenden Elektrode 44 bzw. 45 verbunden sein.

Jedes Elektrodenpaar 44A, 45A; 44A, 45B; 44C, 45C bildet mit der in dem Tank befindlichen (Rest-)Flüssigkeit als Dielektrikum einen Kondensator 46A, 46B, 46C. Die Kapazität jedes Kondensators 46A, 46B, 46C wird fortlaufend gemessen. Die gemessene Kapazität jedes Kondensators 46A, 46B, 46C ist proportional zu dem Flüssigkeitsniveau zwischen den Elektroden 44A, 45A; 44A, 45B; 44C, 45C. Durch die Anordnung und Anzahl der Kondensatoren 46A, 46B, 46C kann in jeder räumlichen Orientierung des Inhalators 10 bzw. der Kartusche bzw. Verbrauchseinheit 17 das Flüssigkeitsniveau im Flüssigkeitstank 18 bestimmt werden.

Aus dem bestimmten Flüssigkeitsniveau im Flüssigkeitstank kann die Restmenge an Flüssigkeit im Flüssigkeitstank 18 bestimmt werden. Aus der bestimmten Flüssigkeits-Restmenge kann beispielsweise eine Voraussage getroffen werden, wann ein Wechsel der Verbrauchseinheit 17 (Kartuschenwechsel) voraussichtlich erforderlich ist, basierend auf dem bisherigen Nutzungsprofil des aktuellen Nutzers.

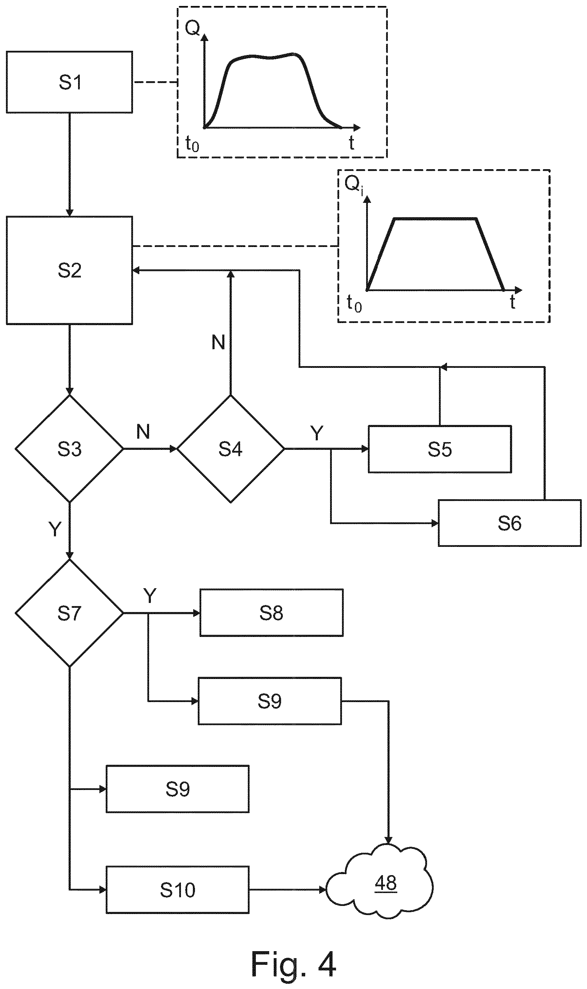

Im Folgenden wird ein bevorzugtes Verfahren zur Steuerung des Inhalators 10 anhand der Figur 4 erläutert.

Im Schritt S1 erfolgt die zeitliche Messung des Volumenstroms Q = dV/dt (siehe Diagramm rechts) der durch den Luftkanal 30 strömenden Luftströmung (bzw. Luft/Dampf-Strömung) mittels der Durchflussmesseinrichtung 37. Gemessen wird der Volumenstrom Q zum jeweils aktuellen Zeitpunkt ta und vorzugsweise das sich daraus ergebende Zugprofil Q(t) über einen gesamten Inhalationszug. Der Volumenstrom Q aufgetragen über der Zeit t für einen gesamten Inhalationszug des Nutzers ergibt ein Zugprofil Q(t), wie beispielsweise im Diagramm rechts von Schritt S1 gezeigt ist.

In dem elektronischen Datenspeicher 35 des Inhalators 10 ist ein ideales Zugprofil Qi(t) gespeichert, siehe Diagramm rechts von Schritt S2.

Im Schritt S2 führt die elektronische Steuerungsvorrichtung 15 eine Vergleichsanalyse durch. Dabei erfolgt ein Vergleich des in Schritt S1 gemessenen aktuellen Volumenstroms Q(t=ta) mit einem entsprechende Idealwert Qi(t=ta), der sich aus dem gespeicherten Zugprofil Qi(t) ergibt, und vorzugsweise ein Vergleich des in Schritt S1 gemessenen Zugprofils Q(t) (insbesondere im Zeitraum t0 entsprechend Zugbeginn bis ta) mit dem gespeicherten idealen Zugprofil Qi(t). Die Vergleichsanalyse umfasst des Weiteren die Bestimmung einer Abweichung des in Schritt S1 gemessenen aktuellen Volumenstroms Q(t=ta) von dem entsprechenden Idealwert Qi(t=ta), und vorzugsweise die Bestimmung einer Abweichung des in Schritt S1 gemessenen Zugprofils Q(t) von dem idealen Zugprofil Qi(t). Im Schritt S2 kann der gemessene aktuelle Volumenstrom Q(t=ta) ein momentaner Wert entsprechend einem einzigen Messwert, oder ein über eine Mehrzahl von Messwerten Q(t=ta1), Q(t=ta2), ... gemittelter Wert sein.

In dem nachfolgenden Schritt S3 wird geprüft, ob der aktuelle Inhalationszug beendet ist. Falls der aktuelle Inhalationszug nicht beendet ist (N), wird im Schritt S4 geprüft, ob die in Schritt S2 bestimmte Abweichung des gemessenen aktuellen bzw. momentanen Volumenstroms Q(t=ta) von dem entsprechenden Idealwert Qi(t=ta) über einem in dem Datenspeicher 35 gespeicherten Schwellwert liegt.

Falls die in dem Schritt S4 betrachtete aktuelle Abweichung über dem gespeicherten Schwellwert liegt (Y), wird in dem Schritt S5 die Wirkstoffabgabe in den Luftstrom, vorzugsweise basierend auf dem Grad der aktuellen Abweichung, durch Ansteuerung der Verdampfungseinrichtung 20 angepasst, insbesondere durch Veränderung des durch das Heizelement 21 fließenden Heizstroms und/oder der Heizdauer des Heizelements 21.

Zusätzlich oder alternativ zu dem Schritt S5 kann in dem Schritt S6 eine Anzeige der fehlerhaften oder nicht idealen momentanen Anwendung des Inhalators 10 an den Nutzer über mindestens ein Signalelement der Signaleinrichtung 19 durch die elektronische Steuerungsvorrichtung 15 veranlasst werden, z.B. die Anzeige einer falschen Luftmenge und/oder Wirkstoffmenge.

Im Anschluss an den Schritt S5 und/oder S6, oder wenn die in dem Schritt S4 betrachtete aktuelle Abweichung unter dem gespeicherten Schwellwert liegt (N), wird der Schritt S2 erneut durchgeführt. Diese Feedback-Schleife wird solange durchgeführt, bis in dem Schritt S3 festgestellt wird, dass der Inhalationszug beendet ist (Y).

Sobald in dem Schritt S3 festgestellt wird, dass der Inhalationszug beendet ist (Y), wird in dem Schritt S7 vorzugsweise geprüft, ob eine Abweichung des in Schritt S1 gemessenen Zugprofils Q(t) von dem idealen Zugprofil Qi(t), jeweils über den gesamten Inhalationszug (gesamte Abweichung), oberhalb eines in dem Datenspeicher 35 gespeicherten Schwellwerts liegt.

Falls in Schritt S7 festgestellt wird, dass die gesamte Abweichung über dem gespeicherten Schwellwert liegt (Y), kann in einem Schritt S8 eine Anzeige der fehlerhaften oder nicht idealen Anwendung des Inhalators 10 an den Nutzer über mindestens ein Signalelement der Signaleinrichtung 19 durch die elektronische Steuerungsvorrichtung 15 veranlasst werden, z.B. die Anzeige einer falschen Luftmenge und/oder Wirkstoffmenge über den betreffenden Inhalationszug.

Zusätzlich oder alternativ zu dem Schritt S8 kann in einem Schritt S9 eine Übertragung der Daten aus der Vergleichsanalyse des Schritts S2 beispielsweise mittels Drahtloskommunikation an einen entfernten Empfänger 48, beispielsweise einen Cloudspeicher, zur weiteren Analyse gesendet werden.

Falls in Schritt S7 festgestellt wird, dass die gesamte Abweichung unter dem gespeicherten Schwellwert liegt (N), kann in einem Schritt S9 eine Anzeige der erfolgreichen Anwendung des Inhalators 10 an den Nutzer über mindestens ein Signalelement der Signaleinrichtung 19 durch die elektronische Steuerungsvorrichtung 15 veranlasst werden, z.B. die Anzeige einer korrekten Luftmenge und/oder Wirkstoffmenge über den betreffenden Inhalationszug.

Zusätzlich oder alternativ zu dem Schritt S9 kann in einem Schritt S10 eine Übertragung der Daten aus der Vergleichsanalyse des Schritts S2 beispielsweise mittels Drahtloskommunikation an einen entfernten Empfänger 48, beispielsweise einen Cloudspeicher, zur weiteren Analyse gesendet werden.

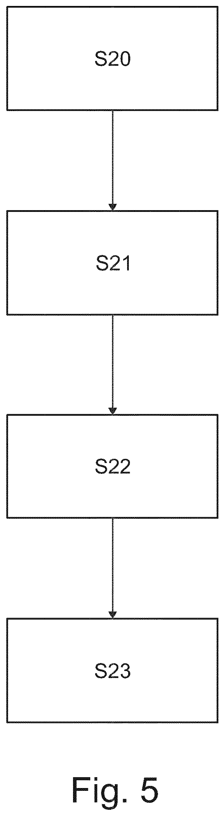

Eine Initialisierungsprozedur für den Inhalator wird im Folgenden anhand von Figur 5 erläutert.

Im Schritt S20 werden Zugprofile über mehrere Inhalationszüge vorzugsweise ohne Wirkstoffabgabe mittels der Durchflussmesseinrichtung 37 gemessen und von der elektronischen Steuerungsvorrichtung 15 erstellt.

In dem Schritt S21 führt die elektronische Steuerungsvorrichtung 15 einen Vergleich der gemessenen Zugprofile mit einem theoretischen Zugprofil durch, das vorteilhaft in dem Datenspeicher 35 gespeichert ist.

In dem Schritt S22 führt die elektronische Steuerungsvorrichtung 15 ein Anfitten (Anpassen) des theoretischen Zugprofils an die gemessenen Zugprofile durch.

In dem Schritt S23 wird das gefittete Zugprofil als ideales (Nutzer-) Zugprofil von der elektronischen Steuerungsvorrichtung 15 in dem Datenspeicher 35 gespeichert, um während der Nutzungsphase für Vergleiche bzw. Vergleichsanalysen zur Verfügung zu stehen.

## Patentansprüche

1. Inhalator (10) umfassend ein Gehäuse (11), einen sich zwischen mindestens einer Lufteinlassöffnung (32) und einer Saugöffnung (24) in dem Gehäuse (11) erstreckenden Luftkanal (30), ein Verabreichungselement (21) zum Vernebeln oder Verdampfen von dem Verabreichungselement (21) zugeführter Flüssigkeit zur Beimischung zu in dem Luftkanal strömender Luft, eine elektronische Steuerungsvorrichtung (15), einen elektronischen Datenspeicher (35) und ein Sensorsystem (33) mit einer Durchflussmessvorrichtung (37) zur Messung des Volumen- und/oder Massenstroms des durch den Luftkanal (30) strömenden Luftstroms, wobei die elektronische Steuerungsvorrichtung (15) zur Erfassung einer Mehrzahl von Luftstrommesswerten über mindestens einen Teil der Dauer eines Inhalationszuges mittels der Durchflussmessvorrichtung (37), zum Vergleich der Mehrzahl von Luftstrommesswerten mit einem in dem Datenspeicher (35) gespeicherten Zugprofil, und zur Ausgabe eines Steuersignals auf der Grundlage des Vergleichs der Mehrzahl von Luftstrommesswerten mit dem gespeicherten Zugprofil eingerichtet ist, wobei das Zugprofil ein definierter idealer zeitlicher Verlauf eines Luft- oder Luft-Dampf-Volumenstroms und/oder -Massenstroms ist, der während wenigstens eines Teils oder während des gesamten idealen Inhalationszuges eines Nutzers durch den Inhalator strömt, **dadurch gekennzeichnet, dass** bei einer definierten Abweichung der Luftstrommesswerte von dem gespeicherten Zugprofil das Steuersignal eine geeignete Maßnahme auslöst, wobei die ausgelöste Maßnahme eine Anpassung der Ansteuerung des Verabreichungselements (21) für die zu verdampfende oder vernebelnde Flüssigkeitsmenge ist.

2. Inhalator (10) nach Anspruch 1, **dadurch gekennzeichnet, dass** die ausgelöste Maßnahme - eine Anpassung der Ansteuerung des Verabreichungselements (21) für die zu verdampfende oder vernebelnde Flüssigkeitsmenge und
- ein Signalisieren einer entsprechenden Information für einen Benutzer mittels einer Signaleinrichtung (19) ist.

3. Inhalator (10) nach einem der vorangehenden Ansprüche, **dadurch gekennzeichnet, dass** die Durchflussmessvorrichtung (37) eine Differenzdruck- oder eine Hitzdraht-Messvorrichtung ist.

4. Inhalator (10) nach Anspruch 3, **dadurch gekennzeichnet, dass** die Differenzdruckmessvorrichtung einen ersten Drucksensor (42) zur Messung des Atmosphärenluftdrucks und einen zweiten Drucksensor (43) zur Messung des in dem Luftkanal (30) herrschenden Luftdrucks aufweist.

5. Inhalator (10) nach einem der vorangehenden Ansprüche, **dadurch gekennzeichnet, dass** das Sensorsystem (33) einen Dampfmengensensor (38) zur Erfassung der während eines Inhalationszuges von einer Verdampfungsvorrichtung (20) abgegebenen Flüssigkeitsmenge aufweist.

6. Inhalator nach Anspruch 5, **dadurch gekennzeichnet, dass** der Dampfmengensensor (38) ein Luftfeuchtesensor ist.

7. Inhalator (10) nach Anspruch 6, **dadurch gekennzeichnet, dass** der Luftfeuchtesensor ein erstes, in dem Luftkanal stromaufwärts von dem Verabreichungselement (21) angeordnetes Feuchtemesselement (40) und ein zweites, in dem Luftkanal stromabwärts von dem Verabreichungselement angeordnetes Feuchtemesselement (41) aufweist.

8. Inhalator (10) nach Anspruch 5, **dadurch gekennzeichnet, dass** der Flüssigkeitsmengensensor (38) ein optischer Absorptionssensor ist.

9. Inhalator (10) nach einem der Ansprüche 5 bis 8, **dadurch gekennzeichnet, dass** die elektronische Steuerungsvorrichtung (15) zur Kompensation eines sich ändernden Wasseranteils in der Flüssigkeit während der Entleerung des Flüssigkeitstanks (18) durch Bestimmung der Restflüssigkeitsmenge im Flüssigkeitstank (18) mittels eines Füllstandssensors (39) oder Abschätzung durch das Zählen der Züge in Verbindung mit der Messung der Zugdauer eingerichtet ist.

10. Inhalator (10) nach einem der Ansprüche 5 bis 9, **dadurch gekennzeichnet, dass** die elektronische Steuerungsvorrichtung (15) zur Berechnung der verdampften Wirkstoffmenge aus der ermittelten verdampften Flüssigkeitsmenge eingerichtet ist.

11. Inhalator (10) nach einem der vorangehenden Ansprüche, **dadurch gekennzeichnet, dass** das Sensorsystem (33) einen Füllstandssensor (39) zur Erfassung einer Restflüssigkeitsmenge in einem Flüssigkeitstank (17) des Inhalators (10) aufweist.

12. Inhalator (10) nach Anspruch 11, **dadurch gekennzeichnet, dass** das der Füllstandssensor (39) ein kapazitiver Sensor ist.

13. Inhalator (10) nach Anspruch 11 oder 12, **dadurch gekennzeichnet, dass** die elektronische Steuerungsvorrichtung (15) zur Feststellung einer Dauer bis zu einem erforderlichen Kartuschenwechsel auf der Grundlage der mittels des Füllstandssensors (39) gemessenen Restflüssigkeitsmenge eingerichtet ist.

14. Inhalator (10) nach einem der vorangehenden Ansprüche, **dadurch gekennzeichnet, dass** die elektronische Steuerungsvorrichtung (15) zur Durchführung einer Initialisierungsprozedur vor der eigentlichen Nutzung mit den folgenden Schritten eingerichtet ist:
- Messung einer Mehrzahl von Luftstrommesswerten über einen oder mehrere Inhalationszüge mittels der Durchflussmessvorrichtung (37);
- Vergleich der Luftstrommesswerte mit einem beispielsweise in dem Datenspeicher (35) gespeicherten theoretischen Zugprofil;
- Bestimmung eines nutzerindividuellen idealen Zugprofils aus dem Vergleich der Luftstrommesswerte mit dem theoretischen Zugprofil; und
- Speichern des nutzerindividuellen idealen Zugprofils in dem Datenspeicher (35).

## Claims

1. Inhaler (10) comprising a housing (11), an air channel (30) extending between at least one air inlet opening (32) and a suction opening (24) in the housing (11), a delivery element (21) for nebulizing or vaporizing liquid, supplied by the delivery element (21), for mixing with air flowing in the air channel, an electronic control device (15), an electronic data memory (35), and a sensor system (33) having a flow measuring device (37) for measuring the volume flow rate and/or mass flow rate of the airflow flowing through the air channel (30), the electronic control device (15) being configured to acquire a plurality of airflow measurements over at least part of the duration of an inhalation puff by means of the flow measuring device (37), to compare the plurality of airflow measurements with a puff profile stored in the data memory (35), and to output a control signal on the basis of the comparison of the plurality of airflow measurements with the stored puff profile, the puff profile being a defined ideal time profile of an air or air-vapor volume flow rate and/or mass flow rate that flows through the inhaler during at least part of or during the entire ideal inhalation puff of a user,
**characterized in that** in the event of a defined deviation of the airflow measurements from the stored puff profile, the control signal triggers a suitable measure, the triggered measure being an adjustment of the actuation of the delivery element (21) for the amount of liquid to be vaporized or nebulized.

2. Inhaler (10) according to claim 1, **characterized in that** the triggered measure is
- adjusting the actuation of the delivery element (21) for the amount of liquid to be vaporized or nebulized and
- signaling relevant information to a user by means of a signaling apparatus (19).

3. Inhaler (10) according to either of the preceding claims,
**characterized in that** the flow measuring device (37) is a differential pressure measuring device or a hot wire measuring device.

4. Inhaler (10) according to claim 3, **characterized in that** the differential pressure measuring device has a first pressure sensor (42) for measuring the atmospheric air pressure and a second pressure sensor (43) for measuring the air pressure prevailing in the air channel (30).

5. Inhaler (10) according to any of the preceding claims,
**characterized in that** the sensor system (33) has a vapor amount sensor (38) for detecting the amount of liquid dispensed by a vaporization device (20) during an inhalation puff.

6. Inhaler according to claim 5, **characterized in that** the vapor amount sensor (38) is a humidity sensor.

7. Inhaler (10) according to claim 6, **characterized in that** the humidity sensor has a first humidity measuring element (40) arranged in the air channel upstream of the delivery element (21) and a second humidity measuring element (41) arranged in the air channel downstream of the delivery element.

8. Inhaler (10) according to claim 5, **characterized in that** the liquid amount sensor (38) is an optical absorption sensor.

9. Inhaler (10) according to any of claims 5 to 8, **characterized in that** the electronic control device (15) is configured to compensate for a changing water content in the liquid during the emptying of the liquid tank (18) by determining the residual amount of liquid in the liquid tank (18) by means of a level sensor (39) or by estimation by counting the puffs in conjunction with measuring the puff duration.

10. Inhaler (10) according to any of claims 5 to 9, **characterized in that** the electronic control device (15) is configured to calculate the amount of active ingredient vaporized from the determined amount of liquid vaporized.

11. Inhaler (10) according to any of the preceding claims,
**characterized in that** the sensor system (33) has a level sensor (39) for detecting a residual amount of liquid in a liquid tank (17) of the inhaler (10).

12. Inhaler (10) according to claim 11, **characterized in that** that the level sensor (39) is a capacitive sensor.

13. Inhaler (10) according to claim 11 or 12, **characterized in that** the electronic control device (15) is configured to ascertain the duration until a cartridge change is required on the basis of the residual amount of liquid measured by means of the level sensor (39).

14. Inhaler (10) according to any of the preceding claims,
**characterized in that** the electronic control device (15) is configured to carry out an initialization procedure prior to actual use comprising the following steps:
- measuring a plurality of airflow measurements over one or more inhalation puffs by means of the flow measuring device (37);
- comparing the airflow measurements with a theoretical puff profile stored, for example, in the data memory (35);
- determining a user-specific ideal puff profile from the comparison of the airflow measurements with the theoretical puff profile; and
- storing the user-specific ideal puff profile in the data memory (35).

## Revendications

1. Inhalateur (10) comprenant un boîtier (11), un conduit d'air (30) s'étendant dans le boîtier (11) entre au moins une ouverture d'entrée d'air (32) et une ouverture d'aspiration (24), un élément d'administration (21) pour nébuliser ou vaporiser un liquide amené à l'élément d'administration (21) pour le mélanger à de l'air s'écoulant dans le conduit d'air, un dispositif de commande électronique (15), une mémoire de données électronique (35) et un système de capteurs (33) avec un dispositif de mesure de débit (37) pour mesurer le débit volumique et/ou massique du flux d'air s'écoulant à travers le conduit d'air (30), le dispositif de commande électronique (15) étant conçu pour acquérir une pluralité de valeurs de mesure de débit d'air sur au moins une partie de la durée d'une bouffée d'inhalation au moyen du dispositif de mesure du débit (37), pour comparer la pluralité de valeurs de mesure de débit d'air à un profil de bouffée enregistré dans la mémoire de données (35) et pour délivrer un signal de commande sur la base de la comparaison de la pluralité de valeurs de mesure de débit d'air avec le profil de bouffée enregistré, le profil de bouffée étant une courbe temporelle idéale définie d'un débit volumique et/ou massique d'air ou d'air-vapeur qui s'écoule à travers l'inhalateur pendant au moins une partie ou pendant toute la bouffée d'inhalation idéale d'un utilisateur, **caractérisé en ce que**, en cas d'écart défini entre les valeurs mesurées de débit d'air et le profil de bouffée enregistré, le signal de commande déclenche une mesure appropriée, la mesure déclenchée étant un ajustement de la commande de l'élément d'administration (21) pour la quantité de liquide à vaporiser ou à nébuliser.

2. Inhalateur (10) selon la revendication 1, **caractérisé en ce que** la mesure déclenchée est
- un ajustement de la commande de l'élément d'administration (21) pour la quantité de liquide à vaporiser ou à nébuliser et
- une signalisation d'une information correspondante à un utilisateur au moyen d'un dispositif de signalisation (19).

3. Inhalateur (10) selon l'une quelconque des revendications précédentes, **caractérisé en ce que** le dispositif de mesure de débit (37) est un dispositif de mesure de pression différentielle ou un dispositif de mesure à fil chaud.

4. Inhalateur (10) selon la revendication 3, **caractérisé en ce que** le dispositif de mesure de pression différentielle comprend un premier capteur de pression (42) pour mesurer la pression atmosphérique et un deuxième capteur de pression (43) pour mesurer la pression d'air régnant dans le conduit d'air (30).

5. Inhalateur (10) selon l'une quelconque des revendications précédentes, **caractérisé en ce que** le système de capteurs (33) comprend un capteur de quantité de vapeur (38) pour détecter la quantité de liquide délivrée par un dispositif de vaporisation (20) pendant une bouffée d'inhalation.

6. Inhalateur selon la revendication 5, **caractérisé en ce que** le capteur de quantité de vapeur (38) est un capteur d'humidité de l'air.

7. Inhalateur (10) selon la revendication 6, **caractérisé en ce que** le capteur d'humidité de l'air comprend un premier élément de mesure de l'humidité (40) disposé dans le conduit d'air en amont de l'élément d'administration (21) et un deuxième élément de mesure de l'humidité (41) disposé dans le conduit d'air en aval de l'élément d'administration.

8. Inhalateur (10) selon la revendication 5, **caractérisé en ce que** le capteur de quantité de liquide (38) est un capteur d'absorption optique.

9. Inhalateur (10) selon l'une quelconque des revendications 5 à 8, **caractérisé en ce que** le dispositif de commande électronique (15) est conçu pour compenser une variation de la teneur en eau dans le liquide pendant la vidange du réservoir de liquide (18) en déterminant la quantité de liquide restante dans le réservoir de liquide (18) au moyen d'un capteur de niveau (39) ou en l'estimant par le comptage des bouffées en combinaison avec la mesure de la durée des bouffées.

10. Inhalateur (10) selon l'une des revendications 5 à 9, **caractérisé en ce que** le dispositif de commande électronique (15) est conçu pour calculer la quantité de principe actif vaporisée à partir de la quantité de liquide vaporisée déterminée.

11. Inhalateur (10) selon l'une quelconque des revendications précédentes, **caractérisé en ce que** le système de capteurs (33) comprend un capteur de niveau de remplissage (39) pour détecter une quantité de liquide restante dans un réservoir de liquide (17) de l'inhalateur (10).

12. Inhalateur (10) selon la revendication 11, **caractérisé en ce que** le capteur de niveau de remplissage (39) est un capteur capacitif.

13. Inhalateur (10) selon la revendication 11 ou 12, **caractérisé en ce que** le dispositif de commande électronique (15) est conçu pour déterminer une durée jusqu'à un changement de cartouche nécessaire sur la base de la quantité de liquide restante mesurée au moyen du capteur de niveau de remplissage (39).

14. Inhalateur (10) selon l'une quelconque des revendications précédentes, **caractérisé en ce que** le dispositif de commande électronique (15) est conçu pour effectuer une procédure d'initialisation avant l'utilisation proprement dite, comprenant les étapes suivantes :
- mesure d'une pluralité de valeurs de mesure de débit d'air sur une ou plusieurs bouffées d'inhalation au moyen du dispositif de mesure de débit (37) ;
- comparaison des valeurs de mesure de débit d'air avec un profil de bouffée théorique enregistré par exemple dans la mémoire de données (35) ;
- détermination d'un profil de bouffée idéal spécifique à l'utilisateur à partir de la comparaison des valeurs de mesure de débit d'air avec le profil de bouffée théorique ; et
- enregistrement du profil de bouffée idéal spécifique à l'utilisateur dans la mémoire de données (35).
